(19) Europäisches Patentamt / European Patent Office / Office européen des brevets

(11) **EP 3 295 876 A1**

(12) **EUROPEAN PATENT APPLICATION**
published in accordance with Art. 153(4) EPC

(43) Date of publication:
**21.03.2018 Bulletin 2018/12**

(51) Int Cl.:
**A61B 8/12** (2006.01)

(21) Application number: **16792594.0**

(22) Date of filing: **28.04.2016**

(86) International application number:
**PCT/JP2016/063441**

(87) International publication number:
**WO 2016/181869 (17.11.2016 Gazette 2016/46)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **13.05.2015 JP 2015098586**

(71) Applicant: **Olympus Corporation**
**Hachioji-shi, Tokyo 192-8507 (JP)**

(72) Inventor: **MIYAKI, Hironaka**
**Hachioji-shi**
**Tokyo 192-8507 (JP)**

(74) Representative: **Gunzelmann, Rainer**
**Wuesthoff & Wuesthoff**
**Patentanwälte PartG mbB**
**Schweigerstraße 2**
**81541 München (DE)**

(54) **ULTRASONIC OBSERVATION DEVICE, OPERATION METHOD FOR ULTRASONIC OBSERVATION DEVICE, AND OPERATION PROGRAM FOR ULTRASONIC OBSERVATION DEVICE**

(57) Provided are: a frequency analysis unit configured to calculate a frequency spectrum of an electrical echo signal received from an ultrasound probe by analyzing a frequency of the echo signal; a band setting unit configured to set, in accordance with a transmission drive wave, a feature calculation-purpose frequency band used in calculating a feature of the frequency spectrum; an intensity correction unit configured to calculate a corrected frequency spectrum by correcting, using a correction amount defined in accordance with the transmission drive wave, intensity of the frequency spectrum in the feature calculation-purpose frequency band set by the band setting unit; and an approximation unit configured to approximate, in the feature calculation-purpose frequency band, the corrected frequency spectrum calculated by the intensity correction unit, thereby to extract a feature of the corrected frequency spectrum. Consequently, provided are an ultrasound observation apparatus capable of keeping a feature of a frequency spectrum corresponding to received ultrasound constant regardless of characteristics of transmitted ultrasound, a method for operating the ultrasound observation apparatus, and a program for operating the ultrasound observation apparatus.

FIG.1

**Description**

Field

The present invention relates to an ultrasound observation apparatus for observing a tissue of an observation target using ultrasound, a method for operating the ultrasound observation apparatus, and a program for operating the ultrasound observation apparatus.

Background

As a technique for observing a tissue of a specimen using ultrasound, there is a known technique for performing a frequency analysis on an electrical echo signal obtained by converting an ultrasound echo from the specimen, and generating a feature image to which a feature of a frequency spectrum obtained as the result of the analysis is added as visual information (for example, refer to Patent Literature 1). In this technique, the feature image and an ultrasound image are sometimes displayed side by side.

The ultrasound image has different display patterns depending on an examination mode. Known examples of the examination mode for ultrasound include: a B mode in which amplitude of an echo signal is converted into luminance to generate an image; a tissue harmonic imaging (THI) mode in which non-linearity of a living body tissue is utilized to generate an image; and a contrast mode in which an ultrasound contrast agent, which is a suspension of microbubbles introduced into an observation target, is highlighted to generate an image (for example, refer to Patent Literature 2).

Citation List

Patent Literature

Patent Literature 1: WO 2012/011414 A
Patent Literature 2: JP 4820494 B

Summary

Technical Problem

When the ultrasound observation is performed, amplitude and a frequency band of transmitted ultrasound are generally changed in accordance with the examination mode. For example, in a case where the ultrasound observation is performed in the contrast mode, ultrasound having lower amplitude and a narrower frequency band than that in the B mode or the THI mode is transmitted. Therefore, computing a feature of a frequency spectrum with the use of the received ultrasound causes such a problem that a calculation result of the feature varies in accordance with the examination mode for the ultrasound image.

The present invention has been made in view of the foregoing, and an object of the invention is to provide an ultrasound observation apparatus capable of keeping a feature of a frequency spectrum corresponding to received ultrasound constant regardless of characteristics of transmitted ultrasound, a method for operating the ultrasound observation apparatus, and a program for operating the ultrasound observation apparatus.

Solution to Problem

In order to solve the above described problem and achieve the object, an ultrasound observation apparatus according to the invention generates an ultrasound image based on an ultrasound echo obtained by an ultrasound probe, the ultrasound probe having an ultrasound transducer configured to transmit ultrasound to an observation target and receive the ultrasound reflected from the observation target. The ultrasound observation apparatus includes: a transmitting and receiving unit configured to: generate an electrical transmission drive wave for generating an ultrasound pulse to be transmitted by the ultrasound probe; output the transmission drive wave to the ultrasound probe; and receive an electrical echo signal from the ultrasound probe; a frequency analysis unit configured to analyze a frequency of the echo signal to calculate a frequency spectrum of the echo signal; a band setting unit configured to set, in accordance with the transmission drive wave, a feature calculation-purpose frequency band that is used in calculating a feature of the frequency spectrum; an intensity correction unit configured to correct intensity of the frequency spectrum in the feature calculation-purpose frequency band set by the band setting unit, using a correction amount defined in accordance with the transmission drive wave, thereby to calculate a corrected frequency spectrum; and an approximation unit configured to approximate the corrected frequency spectrum calculated by the intensity correction unit, in the feature calculation-purpose frequency band, thereby to extract a feature of the corrected frequency spectrum.

In the ultrasound observation apparatus according to the above-described invention, the band setting unit is configured to set the feature calculation-purpose frequency band in accordance with amplitude and a frequency band of the transmission drive wave and characteristics of the ultrasound transducer of the ultrasound probe connected to the ultrasound observation apparatus.

In the ultrasound observation apparatus according to the above-described invention, the band setting unit is configured to set the feature calculation-purpose frequency band by further using information on a reception depth of the ultrasound.

In the ultrasound observation apparatus according to the above-described invention, a plurality of examination modes with different characteristics of transmission drive waves to be generated is settable. The band setting unit is configured to calculate the corrected frequency spectrum by using, as the correction amount, a difference for each frequency between the frequency spectrum of the transmission drive wave in a reference mode included in the plurality of examination modes and the frequency spectrum of the transmission drive wave generated for obtaining the electrical echo signal as a correction target.

In the ultrasound observation apparatus according to the above-described invention, the plurality of examination modes includes a contrast mode for highlighting an ultrasound contrast agent to be introduced into the observation target. The transmission drive wave in the reference mode is a signal having a higher amplitude voltage and a wider frequency band than the transmission drive wave in the contrast mode.

The ultrasound observation apparatus according to the above-described invention further includes an intensity correction information storage unit configured to store intensity correction information required for correcting the intensity of the frequency spectrum. The intensity correction unit is configured to calculate the corrected frequency spectrum using the intensity correction information.

The ultrasound observation apparatus according to the above-described invention further includes: a mode information storage unit configured to store information on a plurality of examination modes that is settable in the ultrasound observation apparatus and is different in characteristics of transmission drive waves to be generated; and a transducer information storage unit configured to store information including the characteristics of the ultrasound transducer of the ultrasound probe that is connectable to the ultrasound observation apparatus. The band setting unit is configured to set the feature calculation-purpose frequency band using the information stored in the mode information storage unit and the information stored in the transducer information storage unit.

The ultrasound observation apparatus according to the above-described invention further includes an attenuation correction unit configured to perform an attenuation correction for reducing a contribution of attenuation occurred in accordance with a reception depth and the frequency of the ultrasound, the attenuation correction being performed before the approximation unit approximates the corrected frequency spectrum and after the band setting unit sets the feature calculation-purpose frequency band, or after the approximation unit approximates the corrected frequency spectrum.

The ultrasound observation apparatus according to the above-described invention further includes: an ultrasound image data generating unit configured to generate ultrasound image data using the echo signal; a feature image data generating unit configured to generate feature image data for displaying information related to the feature; and a display controller configured to cause a display device connected to the ultrasound observation apparatus to display, side by side, two images corresponding to the ultrasound image data and the feature image data.

A method for operating an ultrasound observation apparatus according to the invention is a method for operating an ultrasound observation apparatus for generating an ultrasound image based on an ultrasound echo obtained by an ultrasound probe, the ultrasound probe having an ultrasound transducer configured to transmit ultrasound to an observation target and receive the ultrasound reflected from the observation target. The method includes: a signal generating step of receiving an electrical echo signal from the ultrasound probe, by a transmitting and receiving unit configured to generate an electrical transmission drive wave for generating an ultrasound pulse to be transmitted by the ultrasound probe to output the transmission drive wave to the ultrasound probe; a frequency analysis step of analyzing, by a frequency analysis unit, a frequency of the echo signal to calculate a frequency spectrum of the echo signal; a band setting step of setting, by a band setting unit, a feature calculation-purpose frequency band that is used in calculating a feature of the frequency spectrum, in accordance with the transmission drive wave; an intensity correction step of correcting, by an intensity correction unit, intensity of the frequency spectrum in the feature calculation-purpose frequency band, using a correction amount defined in accordance with the transmission drive wave, thereby to calculate a corrected frequency spectrum; and an approximation step of approximating the corrected frequency spectrum in the feature calculation-purpose frequency band, thereby to extract a feature of the corrected frequency spectrum.

A program for operating an ultrasound observation apparatus according to the invention is a program for operating an ultrasound observation apparatus for generating an ultrasound image based on an ultrasound echo obtained by an ultrasound probe, the ultrasound probe having an ultrasound transducer configured to transmit ultrasound to an observation target and receive the ultrasound reflected from the observation target. The program causes the ultrasound observation apparatus to execute: a signal generating step of receiving an electrical echo signal from the ultrasound probe, by a transmitting and receiving unit configured to generate an electrical transmission drive wave for generating an ultrasound pulse to be transmitted by the ultrasound probe to output the transmission drive wave to the ultrasound probe; a frequency analysis step of analyzing, by a frequency analysis unit, a frequency of the echo signal to calculate a frequency spectrum of the echo signal; a band setting step of setting, by a band setting unit, a feature calculation-purpose frequency band that is used in calculating a feature of the frequency spectrum, in accordance with the transmission drive wave; an intensity correction step of correcting, by an intensity correction unit, intensity of the frequency spectrum in the feature calculation-purpose frequency band, using a correction amount defined in accordance with the transmission drive wave, thereby to calculate a corrected frequency spectrum; and an approximation step of approximating the corrected frequency spectrum in the feature calculation-purpose frequency band, thereby to extract a feature of the corrected frequency spectrum.

Advantageous Effects of Invention

According to the present invention, intensity of a frequency spectrum in a predetermined frequency band is corrected using a correction amount defined in accordance with a transmission drive wave, thereby to calculate a corrected frequency spectrum. After that, the corrected frequency spectrum is approximated in the predetermined frequency band to extract a feature of the corrected frequency spectrum. It is therefore possible to extract the feature independent of the transmission drive wave. Accordingly, the feature of the frequency spectrum corresponding to the received ultrasound can be kept constant regardless of characteristics of transmitted ultrasound.

Brief Description of Drawings

FIG. 1 is a block diagram illustrating a functional configuration of an ultrasound diagnosis system including an ultrasound observation apparatus according to an embodiment of the present invention.
FIG. 2 is a graph schematically illustrating frequency characteristics of sensitivity of an ultrasound transducer.
FIG. 3 is a graph schematically illustrating an exemplary waveform of a transmission drive wave generated by a transmission processing unit of the ultrasound observation apparatus according to the embodiment of the present invention.
FIG. 4 is a graph schematically illustrating a frequency spectrum of the transmission drive wave illustrated in FIG. 3.
FIG. 5 is a graph schematically illustrating another exemplary waveform of the transmission drive wave generated by the transmission processing unit of the ultrasound observation apparatus according to the embodiment of the present invention.
FIG. 6 is a graph schematically illustrating a frequency spectrum of the transmission drive wave illustrated in FIG. 5.
FIG. 7 is a graph illustrating a relation between a reception depth and an amplification factor in an amplification process performed by a reception processing unit of the ultrasound observation apparatus according to the embodiment of the present invention.
FIG. 8 is a graph illustrating a relation between a reception depth and an amplification factor in an amplification correction process performed by an amplification correction unit of the ultrasound observation apparatus according to the embodiment of the present invention.
FIG. 9 is a diagram schematically illustrating a data array in a single sound ray of an ultrasound signal.
FIG. 10 is a graph illustrating an exemplary frequency spectrum calculated by a frequency analysis unit of the ultrasound observation apparatus according to the embodiment of the present invention.
FIG. 11 is a graph illustrating another exemplary frequency spectrum calculated by the frequency analysis unit of the ultrasound observation apparatus according to the embodiment of the present invention.
FIG. 12 is a graph schematically illustrating an intensity correction process performed by an intensity correction unit in a feature calculation-purpose frequency band in the ultrasound observation apparatus according to the embodiment of the present invention.
FIG. 13 is a graph schematically illustrating a process of an approximation unit of the ultrasound observation apparatus according to the embodiment of the present invention.
FIG. 14 is a flowchart illustrating an outline of a process performed by the ultrasound observation apparatus according to the embodiment of the present invention.
FIG. 15 is a flowchart illustrating an outline of a process performed by the frequency analysis unit of the ultrasound observation apparatus according to the embodiment of the present invention.

FIG. 16 is a graph schematically illustrating a waveform of a transmission drive wave according to a modification of the embodiment of the present invention.

FIG. 17 is a graph schematically illustrating a frequency spectrum of the transmission drive wave illustrated in FIG. 16.

FIG. 18 is a graph illustrating a frequency spectrum calculated by a frequency analysis unit when a transmission processing unit generates the transmission drive wave illustrated in FIG. 16, and schematically illustrating an outline of an intensity correction process performed on the frequency spectrum by an intensity correction unit in the ultrasound observation apparatus according to a modification of the embodiment of the present invention. Description of Embodiments

Hereinafter, modes for carrying out the present invention (hereinafter referred to as "embodiment(s)") will be described with reference to the accompanying drawings.

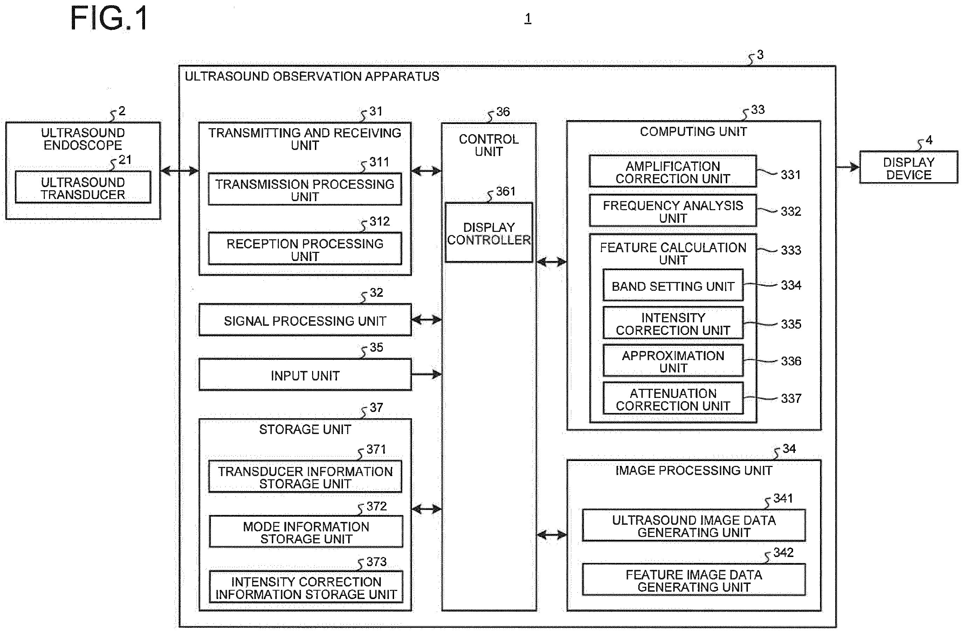

FIG. 1 is a block diagram illustrating a functional configuration of an ultrasound diagnosis system including an ultrasound observation apparatus according to an embodiment of the present invention. An ultrasound diagnosis system 1 illustrated in FIG. 1 includes an ultrasound endoscope 2, an ultrasound observation apparatus 3, and a display device 4. The ultrasound endoscope 2 transmits ultrasound to a subject that is an observation target, and receives the ultrasound reflected from the subject. The ultrasound observation apparatus 3 generates an ultrasound image based on an ultrasound signal obtained by the ultrasound endoscope 2. The display device 4 displays the ultrasound image generated by the ultrasound observation apparatus 3.

In the ultrasound diagnosis system 1, at least any of a B mode, a THI mode, and a contrast mode can be set. In the B mode, amplitude of an echo signal is converted into luminance and displayed. In the THI mode, echo signals are added to or subtracted from each other, to highlight a harmonic component. In the contrast mode, an ultrasound contrast agent introduced into the subject is highlighted. Among them, the setting of the THI mode can be further subdivided in accordance with, for example, resolution and an invasion depth. Generally, modes other than the above-mentioned three modes can also be set. The preset embodiment is based on the assumption that the above-mentioned three modes can be set for convenience of explanation.

The ultrasound endoscope 2 has an ultrasound transducer 21 at a distal end portion thereof. The ultrasound transducer 21 converts an electrical pulse signal received from the ultrasound observation apparatus 3 into an ultrasound pulse (acoustic pulse), and irradiates the subject with the ultrasound pulse. The ultrasound transducer 21 also converts an ultrasound echo reflected from the subject into an electrical echo signal represented by a voltage change, and outputs the electrical echo signal. The ultrasound transducer 21 may be any of a convex transducer, a linear transducer, and a radial transducer. The ultrasound endoscope 2 may be configured to cause the ultrasound transducer 21 to perform scanning mechanically. Alternatively, the ultrasound endoscope 2 may be configured such that a plurality of elements is provided as the ultrasound transducer 21 in the form of an array, and elements related to transmission/reception are switched electronically or a delay is applied to transmission/reception of each element, thereby causing the ultrasound transducer 21 to perform scanning electronically.

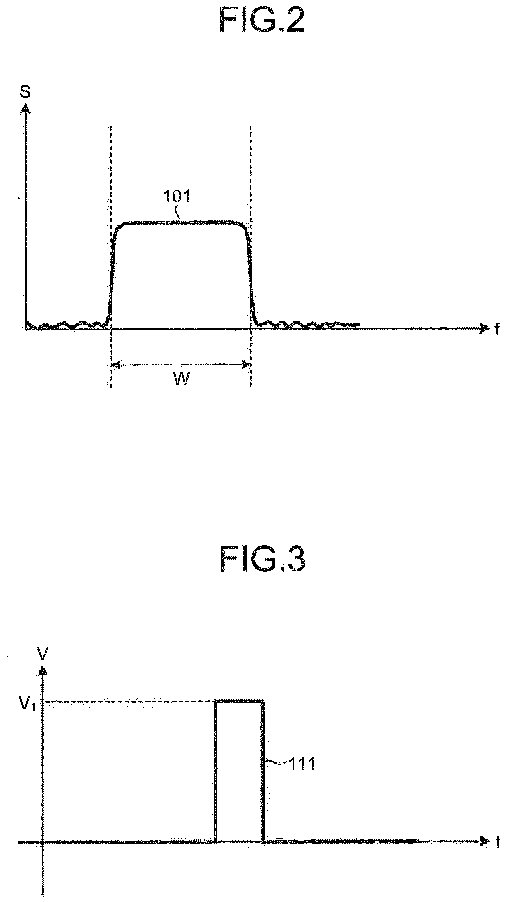

FIG. 2 is a graph schematically illustrating frequency characteristics of sensitivity of the ultrasound transducer 21. In FIG. 2, a horizontal axis represents a frequency f, and a vertical axis represents sensitivity S. As is obvious from a characteristic curve 101 illustrated in FIG. 2, the ultrasound transducer 21 has a high degree of sensitivity with respect to an effective band W that is a specific frequency band, and functions as a filter for the ultrasound signal. Generally, the frequency characteristic of the sensitivity of the ultrasound transducer 21 depends on the type of the ultrasound transducer 21. Therefore, in the ultrasound observation apparatus 3, a transducer information storage unit 371 of a storage unit 37 to be described later stores a sensitivity characteristic corresponding to each kind of the ultrasound transducer 21.

The ultrasound endoscope 2 typically has an imaging optical system and an imaging sensor. The ultrasound endoscope 2 can be inserted into a digestive tract (an esophagus, a stomach, a duodenum, and a large intestine) or a respiratory organ (a trachea and a bronchus) of the subject that is the observation target to capture the digestive tract, the respiratory organ, or peripheral organs thereof (a pancreas, a gallbladder, a bile duct, a biliary tract, a lymph node, a mediastinal organ, and a blood vessel or the like). The ultrasound endoscope 2 also has a light guide that guides illumination light with which the subject is irradiated at the time of the imaging. A distal end portion of the light guide reaches a distal end of an insertion portion of the ultrasound endoscope 2 for the subject, and a proximal end portion of the light guide is connected to a light source device that generates the illumination light.

The ultrasound observation apparatus 3 is electrically connected to the ultrasound endoscope 2, and includes a transmitting and receiving unit 31, a signal processing unit 32, a computing unit 33, an image processing unit 34, an input unit 35, a control unit 36, and a storage unit 37. The transmitting and receiving unit 31 transmits, to the ultrasound transducer 21, a transmission signal (pulse signal) including a high-voltage pulse based on a predetermined waveform and transmission timing, and receives an echo signal that is an electrical reception signal from the ultrasound transducer 21. The signal processing unit 32 generates digital reception data based on the echo signal received from the transmitting and receiving unit 31. The computing unit 33 performs a predetermined calculation on the echo signal received from the transmitting and receiving unit 31. The image processing unit 34 generates various types of image data. The input unit 35 is realized by using a user interface such as a keyboard, a mouse, and a touch panel, and accepts input of various types of information. The control unit 36 comprehensively controls operation of the entire ultrasound diagnosis system 1. The storage unit 37 stores various types of information required for operation of the ultrasound observation apparatus 3.

The transmitting and receiving unit 31 has a transmission processing unit 311 and a reception processing unit 312. The transmission processing unit 311 generates a transmission drive wave that depends on the mode setting for the ultrasound image, and transmits the transmission drive wave to the ultrasound endoscope 2. The reception processing unit 312 performs a sensitivity time control (STC) correction on the echo signal received from the ultrasound endoscope 2. In the STC correction, the greater a distance from the ultrasound transducer 21 to a reflection position of the ultrasound on the observation target, that is, a reception depth of the echo signal, is, the higher an amplification factor that is used for amplification of the echo signal is. In a case where the ultrasound endoscope 2 has such a configuration as to cause the ultrasound transducer 21, i.e. the plurality of elements provided in the form of an array, to perform the scanning electronically, the transmitting and receiving unit 31 has a multichannel circuit for beam composition corresponding to the plurality of elements.

FIG. 3 is a graph schematically illustrating an exemplary waveform of the transmission drive wave generated by the transmission processing unit 311, and illustrating the waveform of the transmission drive wave for the B mode. In FIG. 3, a horizontal axis represents time t, and a vertical axis represents voltage V. A transmission drive wave 111 illustrated in FIG. 3 is a rectangular pulse signal having a maximum voltage (amplitude) $V_1$. The voltage of the transmission drive wave sometimes rises to a negative value in accordance with the kind of the ultrasound transducer 21. In the embodiment, a curve and a straight line representing various waveforms are formed by a set of discrete points.

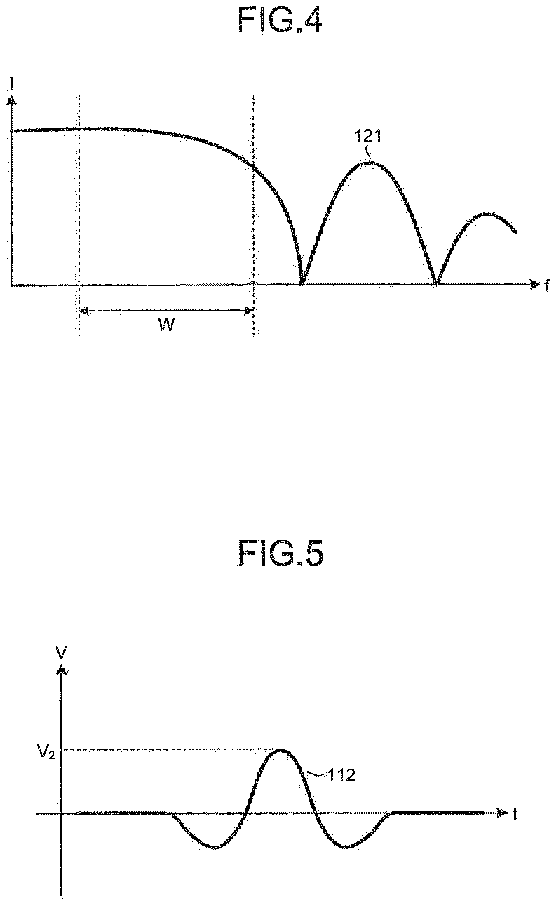

FIG. 4 is a graph schematically illustrating a frequency spectrum of the transmission drive wave for the B mode illustrated in FIG. 3. In FIG. 4, a horizontal axis represents the frequency f, and a vertical axis represents intensity I. A spectrum 121 illustrated in FIG. 4 has such a wideband distribution as to include the effective band W of the ultrasound transducer 21. The "frequency spectrum" as used herein means a "frequency distribution of the intensity" obtained by subjecting the pulse signal to a fast Fourier transform (FFT). The "intensity" as used herein refers to, for example, any of parameters such as the voltage and electric power of the pulse signal, amplitude and time integration values of these parameters, and a combination thereof.

FIG. 5 is a graph schematically illustrating another exemplary waveform of the transmission drive wave generated by the transmission processing unit 311, and illustrating the waveform of the transmission drive wave for the contrast mode. In FIG. 5, a horizontal axis represents the time t, and a vertical axis represents the voltage V. A transmission drive wave 112 illustrated in FIG. 5 is a pulse signal having a maximum voltage (amplitude) $V_2$. The maximum voltage $V_2$ is set smaller than the maximum voltage $V_1$ of the transmission drive wave 111 for the B mode ($V_1 > V_2$).

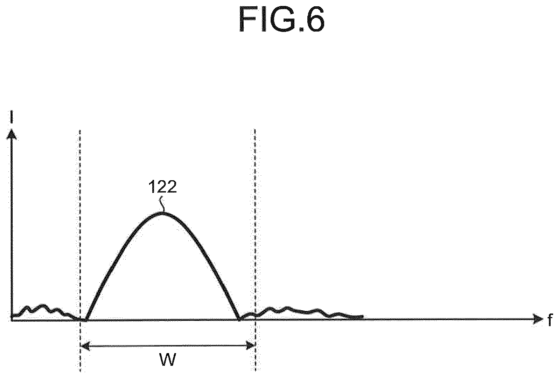

FIG. 6 is a graph schematically illustrating a frequency spectrum of the transmission drive wave for the contrast mode illustrated in FIG. 5. In FIG. 6, a horizontal axis represents the frequency f, and a vertical axis represents the intensity I. A spectrum 122 illustrated in FIG. 6 substantially has the intensity in a band narrower than the effective band W of the ultrasound transducer 21. The transmission drive wave for the contrast mode illustrated in FIGS. 5 and 6 is a signal having low amplitude and including a narrow frequency band as compared with the transmission drive wave for the B mode.

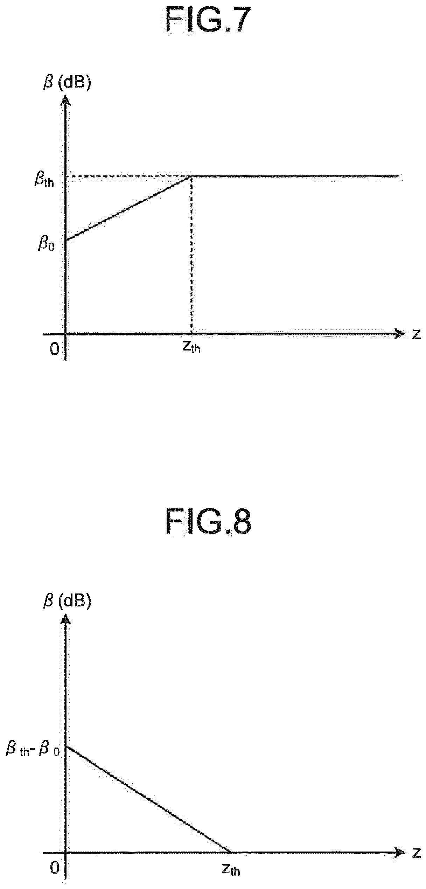

FIG. 7 is a graph illustrating a relation between the reception depth and the amplification factor in the amplification process performed by the reception processing unit 312. The reception depth z illustrated in FIG. 7 is an amount that is computed based on the time that has elapsed since the point of starting to receive ultrasound. As illustrated in FIG. 7, when the reception depth z is less than a threshold value $z_{th}$, the amplification factor $\beta$ (dB) linearly increases from $\beta_0$ to $\beta_{th}$ ($> \beta_0$) as the reception depth z increases. When the reception depth z is equal to or greater than the threshold value $z_{th}$, the amplification factor $\beta$ (dB) is a constant value $\beta_{th}$. The threshold value $z_{th}$ is such a value that the ultrasound signal received from the observation target is attenuated almost completely and noise becomes dominant. More generally, when the reception depth z is less than the threshold value $z_{th}$, the amplification factor $\beta$ is only required to monotonously increase as the reception depth z increases. The relation illustrated in FIG. 7 is stored in the storage unit 37 in advance.

The reception processing unit 312 subjects the amplified echo signal to a process such as filtering, and thereafter to an A/D conversion. The reception processing unit 312 then outputs the echo signal to the signal processing unit 32 and the computing unit 33 as the reception signal.

The transmitting and receiving unit 31 having the above-mentioned functional configuration also has a function of transmitting, to the ultrasound endoscope 2, various control signals output from the control unit 36, and a function of receiving various types of information including an ID for identification from the ultrasound endoscope 2 and transmitting the various types of information to the control unit 36.

The signal processing unit 32 subjects the echo signal to a well-known process such as a bandpass filter, envelope detection, and a logarithmic conversion. The signal processing unit 32 thus generates digital ultrasound image reception data, and outputs the digital ultrasound image reception data to the image processing unit 34. The digital ultrasound image reception data are generated in accordance with the examination mode set in the ultrasound observation apparatus 3. The signal processing unit 32 is realized by using, for example, a general-purpose processor such as a central processing unit (CPU) or a dedicated integrated circuit such as an application specific integrated circuit (ASIC) or a field programmable gate array (FPGA) that executes a specific function.

The computing unit 33 has an amplification correction unit 331, a frequency analysis unit 332, and a feature calculation unit 333. The amplification correction unit 331 performs an amplification correction on the echo signal output from the transmitting and receiving unit 31 so as to make the amplification factor constant regardless of the reception depth. The frequency analysis unit 332 subjects the echo signal that has undergone the amplification correction to the FFT to perform a frequency analysis, thereby computing a plurality of frequency spectra that depends on the reception depth and a reception direction of the ultrasound signal. The feature calculation unit 333 performs a predetermined correction and approximation on the frequency spectrum to compute a feature of the frequency spectrum. The computing unit 33 is realized by using, for example, a general-purpose processor such as a CPU or a dedicated integrated circuit such as an ASIC or an FPGA.

FIG. 8 is a graph illustrating a relation between the reception depth and the amplification factor in the amplification correction process performed by the amplification correction unit 331. As illustrated in FIG. 8, the amplification factor $\beta$ (dB) in the amplification process performed by the amplification correction unit 331 takes a maximum value $\beta_{th} - \beta_0$ when the reception depth z is zero, and linearly decreases as the reception depth z increases from zero to the threshold value $z_{th}$, and becomes zero when the reception depth z is equal to or greater than the threshold value $z_{th}$. The relation illustrated in FIG. 8 is stored in the storage unit 37 in advance. The amplification correction unit 331 performs the amplification correction on the echo signal based on the relation illustrated in FIG. 8, thereby to cancel out the influence of the STC correction performed by the reception processing unit 312 and to output a signal having a constant amplification factor $\beta_{th}$. Needless to say, the relation between the reception depth z and the amplification factor $\beta$ performed by the amplification correction unit 331 varies depending on the relation between the reception depth and the amplification factor in the reception processing unit 312.

The reason for performing the above-mentioned amplification correction will be described. The STC correction is such a correction process as to amplify amplitude of an analog signal waveform uniformly over all frequency bands while causing the amplification factor to monotonously increase with respect to the depth, thereby eliminating the influence of the attenuation from the amplitude of the analog signal waveform. Therefore, in a case where a B mode image, a THI mode image, or a contrast mode image is generated, it is possible to obtain an effect of eliminating the influence of the attenuation from such an image.

On the other hand, in a case where the result of calculating and analyzing the frequency spectrum of the ultrasound is utilized as described in the embodiment, the influence of the attenuation accompanying the propagation of the ultrasound cannot necessarily be accurately eliminated even by the STC correction. This is because an attenuation amount generally depends on the frequency (refer to Expression (1) to be described later) while the amplification factor for the STC correction varies depending on the distance only and does not have the frequency dependence.

One possible way to address such a situation is to output the echo signal that has undergone the STC correction when generating the B mode image or the like, and perform new transmission different from the transmission for generating the B mode image or the like when generating an image that is based on the frequency spectrum, thereby outputting the echo signal that has not undergone the STC correction. However, this solution may lead to reduction in frame rate of image data to be generated based on the echo signal.

In order to address such a situation, in the embodiment, the amplification factor is corrected by the amplification correction unit 331 to eliminate the influence of the STC correction from the signal that has undergone the STC correction for the generation of the B mode image or the like while maintaining the frame rate of the image data to be generated.

The frequency analysis unit 332 samples, at predetermined time intervals, line data of each sound ray subjected to the amplification correction by the amplification correction unit 331, and generates sample data. The frequency analysis unit 332 subjects a sample data group to the FFT process to compute frequency spectra at a plurality of locations (data positons) on the line data.

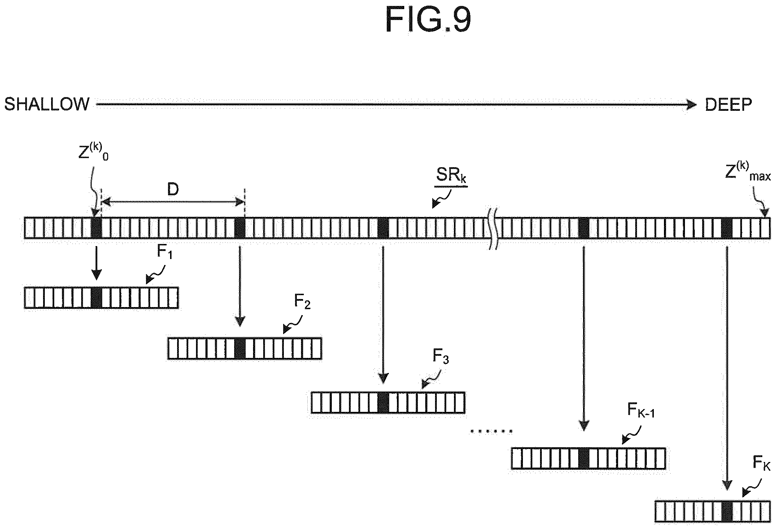

FIG. 9 is a diagram schematically illustrating a data array in a single sound ray of the ultrasound signal. In the sound ray $SR_k$ illustrated in FIG. 9, a white or black rectangle represents a single piece of data at a single sample point. In the sound ray $SR_k$, the closer a piece of data is positioned to the right, the deeper the piece of sample data is located when measured along the sound ray $SR_k$ from the ultrasound transducer 21 (refer to an arrow in FIG. 9). The sound ray $SR_k$ is discretized at time intervals corresponding to a sampling frequency (for example, 50 MHz) in the A/D conversion performed by the transmitting and receiving unit 31. In FIG. 9, an eighth data position on the sound ray $SR_k$ of a number k is set as an initial value $Z^{(k)}_0$ in a direction of the reception depth z. However, the position of the initial value can be arbitrarily set. The calculation result of the frequency analysis unit 332 is obtained as a complex number and stored in the storage unit 37.

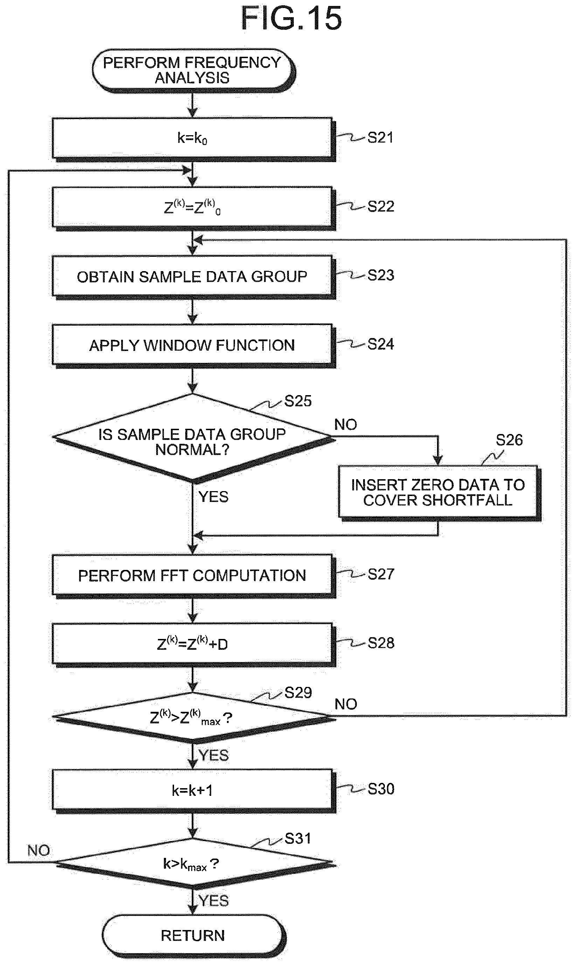

Data groups $F_j$ (j = 1, 2, ..., K) illustrated in FIG. 9 are the sample data groups to be subjected to the FFT process. Generally, the number of pieces of data in the sample data group needs to be a power of two in order to perform the FFT process. In this sense, the sample data groups $F_j$ (j = 1, 2, ..., K-1) are normal data groups since the number of pieces of data is 16 (i.e. $2^4$). However, the sample data group $F_K$ is an abnormal data group since the number of pieces of data is 12. When the FFT process is performed on the abnormal data group, a process for generating a normal sample data group is performed by inserting zero data to cover shortfall. This process will be described in detail later when a process of the frequency analysis unit 332 is described (refer to FIG. 15).

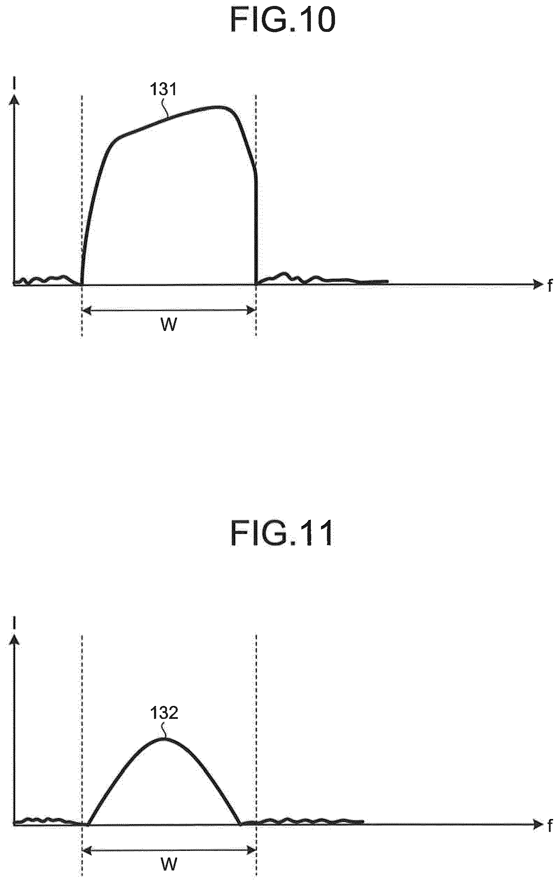

FIG. 10 is a graph illustrating an exemplary frequency spectrum calculated by the frequency analysis unit 332, and schematically illustrating a waveform of the echo signal generated by the reception processing unit 312 for the transmission drive wave for the B mode illustrated in FIG. 4. FIG. 11 is a graph illustrating another exemplary frequency spectrum calculated by the frequency analysis unit 332, and schematically illustrating a waveform of the echo signal received by the reception processing unit 312 for the transmission drive wave for the contrast mode illustrated in FIG. 6. The frequency spectrum calculated by the frequency analysis unit 332 represents a "frequency distribution of the intensity I at a certain reception depth z" obtained by subjecting the sample data group to the FFT process. The "intensity" as used herein refers to, for example, any of parameters such as voltage of the echo signal, electric power of the echo signal, a sound pressure of the ultrasound echo, and acoustic energy of the ultrasound echo, amplitude and time integration values of these parameters, and a combination thereof.

In FIGS. 10 and 11, a horizontal axis represents the frequency f, and a vertical axis represents a common logarithm (decibel representation) of an amount obtained by dividing intensity $I_0$ by reference intensity $I_c$ (constant), that is, $I = 10\log_{10}(I_0/I_c)$. Spectra 131 and 132 illustrated respectively in FIGS. 10 and 11 are frequency distributions at the same reception depth z.

Generally, in a case where the observation target is a living body tissue, the frequency spectrum of the echo signal shows a tendency that depends on behavior of the living body tissue scanned with the ultrasound. This is because the frequency spectrum of the echo signal correlates with the size, number density, and acoustic impedance or the like of a scatterer that scatters the ultrasound. The "behavior of the living body tissue" as used herein represents, for example, a malignant tumor (cancer), a benign tumor, an endocrine tumor, a mucinous tumor, a normal tissue, a cyst, and a vascular channel or the like.

The feature calculation unit 333 has a band setting unit 334, an intensity correction unit 335, an approximation unit 336, and an attenuation correction unit 337. The band setting unit 334 sets a frequency band for computing the feature. The intensity correction unit 335 corrects the intensity of the frequency spectrum in the frequency band set by the band setting unit 334. The approximation unit 336 approximates the frequency spectrum subjected to the intensity correction (corrected frequency spectrum) by means of a regression analysis. The attenuation correction unit 337 corrects, for the approximated corrected frequency spectrum, the influence of the attenuation of the ultrasound that depends on the reception depth and the frequency of the ultrasound.

The band setting unit 334 sets a feature calculation-purpose frequency band based on, for example, the amplitude and an effective frequency band of the transmission drive wave and the frequency characteristic of the sensitivity of the ultrasound transducer 21. The effective frequency band of the transmission drive wave as used herein represents, for example, a frequency band in which the intensity of the transmission drive wave is greater than a predetermined threshold value. The feature calculation-purpose frequency band represents a frequency band on which the approximation is performed by the approximation unit 336, and in which a good S/N ratio and sufficient signal intensity can be obtained. The band setting unit 334 may set, as the feature calculation-purpose frequency band, an area in which the intensity of the echo signal generated based on the received echo signal is equal to or greater than a predetermined threshold value.

Furthermore, the band setting unit 334 may set the feature calculation-purpose frequency band by further using information of the reception depth that is the distance from the surface of the ultrasound transducer 21. Specifically, for the feature calculation-purpose frequency band set as mentioned above, the band setting unit 334 sets, as a final feature calculation-purpose frequency band, the frequency band corrected such that the greater the reception depth is, the narrower a bandwidth is and the smaller a maximum frequency in the band is. This is because the ultrasound has such a characteristic that a high frequency component attenuates fast. In a case where the information of the reception depth is further used, a relation between the reception depth and a correction amount for the frequency band is stored in the storage unit 37 in advance, and the band setting unit 334 only needs to refer to the storage unit 37 to perform the correction.

For the frequency spectrum calculated by the frequency analysis unit 332, the intensity correction unit 335 corrects, on a frequency basis, the intensity in the feature calculation-purpose frequency band set by the band setting unit 334. The intensity correction unit 335 corrects, on a frequency basis, the intensity of the frequency spectrum in the feature calculation-purpose frequency band by referring to intensity correction information stored in an intensity correction information storage unit 373 of the storage unit 37 to be described later. The intensity correction unit 335 thus computes the corrected frequency spectrum. The intensity correction information will be described in detail when the intensity correction information storage unit 373 is described.

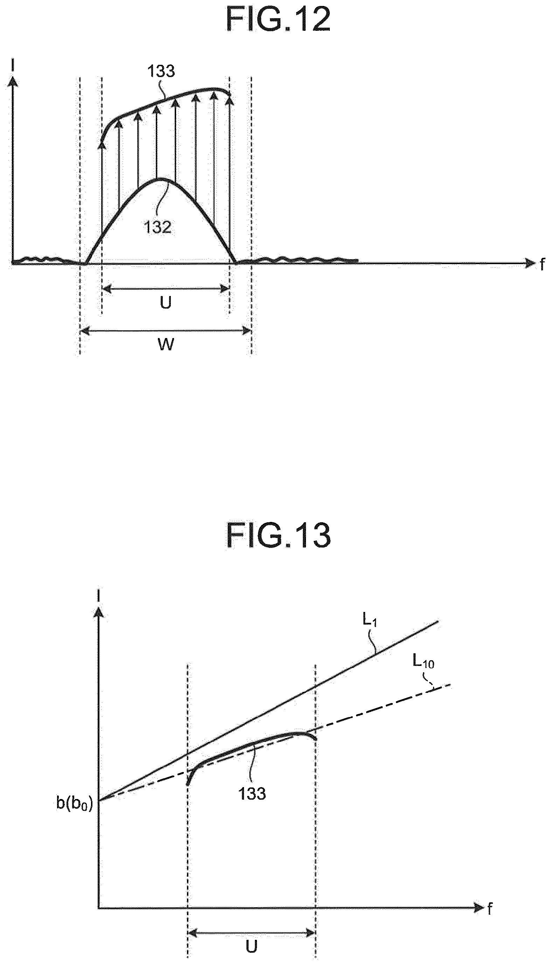

FIG. 12 is a graph schematically illustrating the intensity correction process performed by the intensity correction unit 335 in a feature calculation-purpose frequency band U. Specifically, FIG. 12 is the diagram schematically illustrating a case where the intensity correction unit 335 performs the intensity correction process on the spectrum 132 illustrated in FIG. 11 on the assumption that a reference mode is the B mode and a correction target is the contrast mode. Arrows illustrated in FIG. 12 schematically represent corrections in typical frequencies. A correction spectrum 133 obtained by means of the correction has the same shape as the spectrum 131 illustrated in FIG. 10 in the frequency band U.

The approximation unit 336 performs the regression analysis on the corrected frequency spectrum after the intensity correction in the feature calculation-purpose frequency band set by the band setting unit 334, and approximates the corrected frequency spectrum by means of a primary expression. Consequently, the approximation unit 336 extracts a feature of the corrected frequency spectrum. For example, in a case of the correction spectrum 133 illustrated in FIG. 12, the approximation unit 336 obtains an approximate straight line by performing the regression analysis in the frequency band U. FIG. 13 is a graph schematically illustrating the above-mentioned process of the approximation unit 336. A straight line $L_{10}$ illustrated in FIG. 13 is an approximate straight line calculated by the approximation unit 336. When the approximate straight line $L_{10}$ is represented by a primary expression of the frequency f, that is, $I = a_0 f + b_0$, the approximation unit 336 extracts, as features corresponding to the straight line $L_{10}$, a slope $a_0$, an intercept $b_0$, and a mid-band fit $c_0 = a_0 f_M + b_0$ that is a value of the intensity I in a center frequency $f_M = (f_s + f_e)/2$ of the frequency band U. In the embodiment, after that, the attenuation correction unit 337 performs the attenuation correction on the features $a_0$, $b_0$, and $c_0$, thereby computing a final feature. Hereinafter, therefore, the feature of the corrected frequency spectrum extracted by the approximation unit 336 is referred to as a "temporary feature". Note that the approximation unit 336 can also approximate the frequency spectrum by means of a polynomial expression including a secondary or higher-order expression using the regression analysis.

Among the three temporary features, the slope $a_0$ correlates with the size of the scatterer of the ultrasound. It is generally considered that the greater the scatterer is the smaller the value of the slope is. The intercept $b_0$ correlates with the size of the scatterer, the difference in the acoustic impedance, and the number density (concentration) of the scatterer or the like. Specifically, it is considered that the greater the scatterer is the greater the value of the intercept $b_0$ is, the greater the difference in the acoustic impedance is the greater the value of the intercept $b_0$ is, and the greater the number density of the scatterer is the greater the value of the intercept $b_0$ is. The mid-band fit $c_0$ is an indirect parameter derived from the slope $a_0$ and the intercept $b_0$, and gives the intensity of the spectrum in the center of the effective frequency band. Therefore, it is considered that the mid-band fit $c_0$ correlates with the luminance of the B mode image to some extent in addition to the size of the scatterer, the difference in the acoustic impedance, and the number density of the scatterer.

The attenuation correction unit 337 extracts the feature by performing the attenuation correction on the temporary features (slope $a_0$, intercept $b_0$, and mid-band fit $c_0$) extracted by the approximation unit 336. Hereinafter, the correction performed by the attenuation correction unit 337 will be described. Generally, an attenuation amount A (f, z) of the ultrasound is represented by

$$A\ (f,\ z)\ =\ 2\alpha z f\ \ldots\ (1).$$

In the expression, $\alpha$ is an attenuation factor, z is the reception depth of the ultrasound, and f is the frequency. As is obvious from Expression (1), the attenuation amount A (f, z) is proportional to the frequency f. If an observation target is a living body, a specific value of the attenuation factor $\alpha$ is 0.0 to 1.0 (dB/cm/MHz) and more preferably 0.3 to 0.7 (dB/cm/MHz), which is defined in accordance with a portion of the living body. For example, in a case where the observation target is a pancreas, $\alpha$ might be defined as $\alpha = 0.6$ (dB/cm/MHz). In the embodiment, the value of the attenuation factor $\alpha$ may be able to be set or changed in response to input from the input unit 35.

The attenuation correction unit 337 extracts the feature by performing the attenuation correction on the slope $a_0$, the intercept $b_0$, and the mid-band fit $c_0$ in the following manner.

$$a\ =\ a_0\ +\ 2\alpha z\ \ldots\ (2)$$

$$b\ =\ b_0\ \ldots\ (3)$$

$$c\ =\ c_0\ +\ 2\alpha z f_M\ (\ =\ a f_M\ +\ b)\ \ldots\ (4)$$

As is obvious from Expressions (2) and (4), the greater the reception depth z of the ultrasound is, the greater the correction amount for the correction performed by the attenuation correction unit 337 is. According to Expression (3), the correction for the intercept is an identity transformation. This is because the intercept is a frequency component corresponding to the frequency 0 (Hz), and is not affected by the attenuation.

An expression of a straight line having the slope a in Expression (2) and the intercept b in Expression (3) is given by

$$I = af + b = (a_0 + 2\alpha z) f + b_0 \ldots \quad (5).$$

A straight line $L_1$ illustrated in FIG. 13 schematically represents this approximate straight line. As is obvious from Expression (5) and FIG. 13, the straight line $L_1$ has the greater slope and the same intercept as compared with the straight line $L_{10}$ before the attenuation correction.

As the feature calculated by the feature calculation unit 333 having the above-mentioned functional configuration, it is possible to employ a statistic of slopes a, intercepts b, and mid-band fits c calculated by the attenuation correction unit 337 in a plurality of unit areas (also referred to as discrimination windows). Examples of such a statistic include an average, a standard deviation, a variance, and entropy.

The image processing unit 34 has an ultrasound image data generating unit 341 and a feature image data generating unit 342. The ultrasound image data generating unit 341 generates, from the echo signal, ultrasound image data in a selected display mode. The feature image data generating unit 342 generates feature image data that display visual information related to the feature calculated by the feature calculation unit 333.

The ultrasound image data generating unit 341 generates the ultrasound image data that depend on the set examination mode using the echo signal received from the signal processing unit 32. In the embodiment, the ultrasound image data generating unit 341 generates any of B mode image data, THI mode image data, and contrast mode image data.

The feature image data generating unit 342 generates the feature image data indicating the feature calculated by the feature calculation unit 333. Specifically, the feature image data generating unit 342 generates the feature image data by superimposing the visual information related to the feature on each pixel of an image in the ultrasound image data. Examples of the visual information related to the feature include variables of a color space constituting a predetermined color system such as a color phase, colorfulness, brightness, a luminance value, red (R), green (G), and blue (B). A feature image may be a two-dimensional image or may be a three-dimensional image.

The feature image data generating unit 342 assigns the visual information corresponding to the feature of the frequency spectrum calculated from the sample data group $F_j$, to a pixel area corresponding to a data amount of a single amplitude data group $F_j$ ($j = 1, 2, ..., K$) illustrated in FIG. 9, for example.

The feature image data generating unit 342 may generate the feature image data, for example, by associating the visual information with any one of the above-mentioned slope a, intercept b, and mid-band fit c. The feature image data generating unit 342 may be configured to generate the feature image data by associating the color phase with one of two features selected from among the slope a, the intercept b, and the mid-band fit c, and associating light and darkness with the other.

The control unit 36 has a display controller 361 that controls the display of the display device 4. The display controller 361 performs control to cause the display device 4 to display, side by side, an ultrasound image corresponding to the ultrasound image data generated by the ultrasound image data generating unit 341 and a feature image corresponding to the feature image data generated by the feature image data generating unit 342.

The control unit 36 is realized by using, for example, a general-purpose processor such as a CPU having a calculation and control function or a dedicated integrated circuit such as an ASIC or an FPGA. In a case where the control unit 36 is realized by the general-purpose processor or the FPGA, the control unit 36 reads, from the storage unit 37, various programs and various types of data stored in the storage unit 37 to execute various calculation processes related to a method for operating the ultrasound observation apparatus 3. The control unit 36 thus comprehensively controls the ultrasound observation apparatus 3. In a case where the control unit 36 is configured using the ASIC, the control unit 36 may execute the various processes independently, or may execute the various processes by using the various types of data stored in the storage unit 37. The control unit 36 can also be configured using a general-purpose processor or a dedicated integrated circuit that is common to the signal processing unit 32 and the computing unit 33.

The storage unit 37 has the transducer information storage unit 371, a mode information storage unit 372, and the intensity correction information storage unit 373. The transducer information storage unit 371 stores information such as a sensitivity band as transducer information unique to the ultrasound transducer 21 connected to the ultrasound observation apparatus 3. The mode information storage unit 372 stores information about the examination mode that can be set. The intensity correction information storage unit 373 stores the intensity correction information for each examination mode.

The transducer information storage unit 371 stores the sensitivity characteristic that depends on the kind of the ultrasound transducer 21. The sensitivity characteristic is given, for example, by the characteristic curve 101 illustrated in FIG. 2.

The mode information storage unit 372 stores a parameter that depends on the examination mode that can be set. An example of the parameter includes a parameter about the transmission drive wave. More specifically, the mode information storage unit 372 stores information of the amplitude and the frequency spectrum of the transmission drive wave generated for each examination mode.

The intensity correction information storage unit 373 stores, with respect to the reference mode defined in advance among the examination modes that can be set, information for correcting the intensity of another examination mode. For example, the intensity correction information storage unit 373 regards, as the reference mode, the B mode in which the transmission drive wave having the maximum voltage of high amplitude and including the frequency spectrum in a wide frequency band is generated. The intensity correction information storage unit 373 then stores, as the intensity correction information, a difference from another examination mode (THI mode, contrast mode or the like) in each frequency of the frequency spectrum of the transmission drive wave.

The storage unit 37 also stores, in addition to the above-mentioned items, for example, information required for the amplification process (relation between the amplification factor and the reception depth illustrated in FIG. 7), information required for the amplification correction process (relation between the amplification factor and the reception depth illustrated in FIG. 8), information required for the attenuation correction process (refer to Expression (1)), and information of a window function (such as Hamming, Hanning, and Blackman) required for the frequency analysis process.

The storage unit 37 also stores various programs including an operation program for executing the method for operating the ultrasound observation apparatus 3. The operation program can also be recorded in a computer-readable recording medium such as a hard disc, a flash memory, a CD-ROM, a DVD-ROM, and a flexible disc to be distributed widely. The above-mentioned various programs can also be downloaded and obtained via a communication network. The communication network as used herein is realized by, for example, whether wired or wireless, an existing public line network, a local area network (LAN), and a wide area network (WAN) or the like.

The storage unit 37 having the above-mentioned configuration is realized by using, for example, a read only memory (ROM) on which the various programs or the like are installed in advance, and a random access memory (RAM) that stores calculation parameters and data or the like for each process.

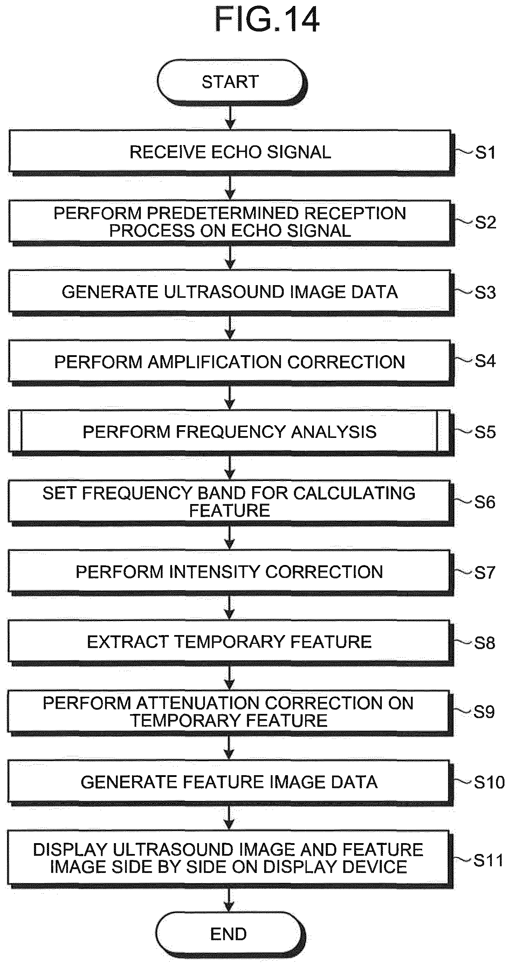

FIG. 14 is a flowchart illustrating an outline of the process performed by the ultrasound observation apparatus 3. The flowchart illustrated in FIG. 14 represents the process after the transmission processing unit 311 starts to transmit the transmission drive wave that depends on the examination mode, and the ultrasound transducer 21 starts to transmit the ultrasound.

First, the reception processing unit 312 receives, from the ultrasound endoscope 2, the echo signal that is the result of measurement of the observation target obtained by the ultrasound transducer 21 (step S1).

The reception processing unit 312 that has received the echo signal from the ultrasound transducer 21 performs the predetermined reception process on the echo signal (step S2). Specifically, the reception processing unit 312 subjects the echo signal to the amplification (STC correction), and thereafter to the process such as the filtering and the A/D conversion. When the reception processing unit 312 performs the amplification, for example, the relation between the amplification factor and the reception depth illustrated in FIG. 7 is used.

Next, the ultrasound image data generating unit 341 generates the ultrasound image data using the echo signal subjected to the process such as the amplification by the reception processing unit 312, and outputs the ultrasound image data to the display device 4 (step S3). In this case, the display controller 361 may perform the control to cause the display device 4 to display the ultrasound image data.

The amplification correction unit 331 performs the amplification correction on the echo signal output from the transmitting and receiving unit 31 so that the amplification factor is constant regardless of the reception depth (step S4). At this time, the amplification correction unit 331 performs the amplification correction based on, for example, the relation between the amplification factor and the reception depth illustrated in FIG. 8.

After that, the frequency analysis unit 332 performs, by means of the FFT, the frequency analysis on the echo signal of each sound ray after the amplification correction, thereby computing the frequency spectra for all the sample data groups, and stores the frequency spectra in the storage unit 37 (step S5). FIG. 15 is a flowchart illustrating an outline of the process performed by the frequency analysis unit 332 in step S5. Hereinafter, the frequency analysis process will be described in detail with reference to the flowchart illustrated in FIG. 15.

First, the frequency analysis unit 332 sets a counter $k$ identifying a sound ray to be analyzed to $k_0$ (step S21).

Next, the frequency analysis unit 332 sets an initial value $Z^{(k)}_0$ of a data position (corresponding to the reception depth) $Z^{(k)}$ representing a series of data groups (sample data groups) generated for FFT computation (step S22). As mentioned above, in FIG. 9, the eighth data position on the sound ray $SR_k$ is set as the initial value $Z^{(k)}_0$, for example

After that, the frequency analysis unit 332 obtains the sample data group (step S23), and applies the window function stored in the storage unit 37 to the obtained sample data group (step S24). By applying the window function to the sample data group in the above-mentioned manner, it is possible to avoid discontinuity of the sample data group at the boundary and prevent generation of an artifact.

Next, the frequency analysis unit 332 determines whether the sample data group with the data position $Z^{(k)}$ is a normal data group (step S25). As described with reference to FIG. 9, the number of pieces of data in the sample data group needs to be a power of two. Hereinafter, the number of pieces of data in the normal sample data group is assumed to be $2^n$ (n is a positive integer). In the embodiment, the data position $Z^{(k)}$ is set so as to be located, as much as possible, in the center of the sample data group to which the data position $Z^{(k)}$ belongs. Specifically, since the number of pieces of data in the sample data group is $2^n$, the data position $Z^{(k)}$ is set at a $2^n/2$ (i.e. $2^{n-1}$)-th position near the center of the sample data group. In this case, it is meant by the normal sample data group that $2^{n-1} - 1$ (i.e. assumed to be N) pieces of data are present on a shallow side beyond the data position $Z^{(k)}$, and $2^{n-1}$ (i.e. assumed to be M) pieces of data are present on a deep side beyond the data position $Z^{(k)}$. In the case illustrated in FIG. 9, the sample data group $F_j$ (j = 1, 2, ..., K-1) is normal. In the example illustrated in FIG. 9, n = 4 (N = 7, M = 8) is satisfied.

When the sample data group with the data position $Z^{(k)}$ is normal as the result of the determination in step S25, (step S25: Yes), the frequency analysis unit 332 proceeds to step S27 to be described later.

When the sample data group with the data position $Z^{(k)}$ is not normal as the result of the determination in step S25, (step S25: No), the frequency analysis unit 332 generates a normal sample data group by inserting zero data to cover shortfall (step S26). To the sample data group determined to be not normal in step S25 (for example, the sample data group $F_K$ in FIG. 9), the window function is applied before the zero data are added. Therefore, the discontinuity of the data does not occur even when the zero data are inserted into the sample data group. After step S26, the frequency analysis unit 332 proceeds to step S27 to be described later.

In step S27, the frequency analysis unit 332 performs the FFT computation using the sample data group to obtain the frequency spectrum which is the frequency distribution of the amplitude (step S27).

Next, the frequency analysis unit 332 varies the data position $Z^{(k)}$ with a step width D (step S28). The step width D is stored in the storage unit 37 in advance. In the example illustrated in FIG. 9, D = 15 is satisfied. The step width D desirably coincides with a data step width that is utilized when the ultrasound image data generating unit 341 generates the B mode image data. In a case where a calculation amount in the frequency analysis unit 332 needs to be reduced, a value greater than the data step width may be set as the step width D.

After that, the frequency analysis unit 332 determines whether the data position $Z^{(k)}$ is greater than a maximum value $Z^{(k)}_{max}$ in the sound ray $SR_k$ (step S29). When the data position $Z^{(k)}$ is greater than the maximum value $Z^{(k)}_{max}$ (step S29: Yes), the frequency analysis unit 332 increases the counter k by one (step S30). This means that the process is transferred to the next sound ray. On the other hand, when the data position $Z^{(k)}$ is equal to or less than the maximum value $Z^{(k)}_{max}$ (step S29: No), the frequency analysis unit 332 returns to step S23.

After step S30, the frequency analysis unit 332 determines whether the counter k is greater than a maximum value $k_{max}$ (step S31). When the counter k is greater than $k_{max}$ (step S31: Yes), the frequency analysis unit 332 ends a series of frequency analysis processes. On the other hand, when the counter k is equal to or less than $k_{max}$ (step S31: No), the frequency analysis unit 332 returns to step S22. The maximum value $k_{max}$ is a value indicated in an instruction arbitrarily input by a user through the input unit 35, or a value set in the storage unit 37 in advance.

In the above-mentioned way, the frequency analysis unit 332 performs multiple FFT computations on ($k_{max}$ - $k_0$ + 1) sound rays within the area to be analyzed. The frequency spectrum obtained as the result of the FFT computation is stored in the storage unit 37 together with the reception depth and the reception direction.

In the above-mentioned description, the frequency analysis unit 332 performs the frequency analysis process on all the areas in which the ultrasound signals have been received. Alternatively, the input unit 35 can be configured to be able to accept input for setting a partial area outlined in accordance with a specific depth range and sound ray range, and the frequency analysis process can be performed only within the set partial area.

Subsequent to the above-mentioned frequency analysis process in step S5, the band setting unit 334 sets a frequency band for calculating the feature (step S6). By step S6, the frequency band U illustrated in FIG. 12 is set, for example.

After that, the intensity correction unit 335 refers to the intensity correction information stored in the intensity correction information storage unit 373, and corrects the intensity of the frequency spectrum in the feature calculation-purpose frequency band (step S7). For example, in a case where the B mode is regarded as the reference mode, when the contrast mode is set as the examination mode, the intensity correction unit 335 corrects the intensity of the spectrum 132 as illustrated in FIG. 12 to obtain the correction spectrum 133.

The approximation unit 336 performs the regression analysis on the corrected frequency spectrum subjected to the intensity correction in the feature calculation-purpose frequency band, thereby approximating the corrected frequency spectrum by means of the primary expression. Consequently, the approximation unit 336 extracts the temporary feature that is the feature of the corrected frequency spectrum (step S8). Examples of the temporary feature that is extracted in this step include the above-mentioned slope $a_0$, intercept $b_0$, and mid-band fit $c_0$. A straight line having these temporary features can be exemplified by the straight line $L_{10}$ illustrated in FIG. 13.

The attenuation correction unit 337 performs the attenuation correction on the temporary feature to extract the feature (step S9). For example, the attenuation correction unit 337 performs the attenuation correction using Expressions (2) to (4) mentioned above to extract the slope a, the intercept b, and the intensity c as the features. A straight line having these features can be exemplified by the straight line $L_1$ illustrated in FIG. 13.

The feature image data generating unit 342 generates the feature image data using the feature extracted in step S9 (step S10). For example, the feature image data generating unit 342 generates the feature image data by superimposing the visual information (e.g. color phase) associated with the feature on each pixel in the ultrasound image data, thereby generating the feature image data. The feature image data generating unit 342 transmits the generated feature image data to the display device 4.

After that, the display controller 361 performs the control to cause the display device 4 to display, side by side on a screen, the ultrasound image corresponding to the ultrasound image data generated in step S3 and the feature image corresponding to the feature image data generated in step S10 (step S11).

After step S11, the ultrasound observation apparatus 3 ends a series of processes. The ultrasound observation apparatus 3 repeatedly executes the processes in steps S1 to S11.

According to the embodiment of the present invention described above, the intensity of the frequency spectrum in the predetermined frequency band is corrected using the correction amount defined in accordance with the transmission drive wave, whereby the corrected frequency spectrum is computed. After that, the corrected frequency spectrum is approximated in the predetermined frequency band, whereby the feature of the corrected frequency spectrum is extracted. Therefore, the feature that is independent of the transmission drive wave can be extracted. Thus, the feature of the frequency spectrum corresponding to the received ultrasound can be kept constant regardless of the characteristic of the transmitted ultrasound.

In addition, according to the embodiment, when the ultrasound image and the feature image are displayed side by side, the feature image does not vary in accordance with the examination mode. Therefore, a user such as a doctor can confirm the feature image under the same condition even when the examination mode is changed. In particular, according to the embodiment, the feature image does not vary even when the examination mode is switched from the B mode or the THI mode, in which the characteristic of the transmission drive wave is significantly different, to the contrast mode. Therefore, the user can visually check the feature image without feeling a sense of discomfort. As a result, the user can easily perform the diagnosis using the feature image, which can contribute to an improvement in accuracy of the diagnosis.

Moreover, according to the embodiment, in a case where the frequency band is set by further using the information of the reception depth of the ultrasound, the frequency band in which the S/N ratio has been deteriorated due to the attenuation of the ultrasound is not included in the approximation, whereby accuracy of the computation of the feature can be improved.

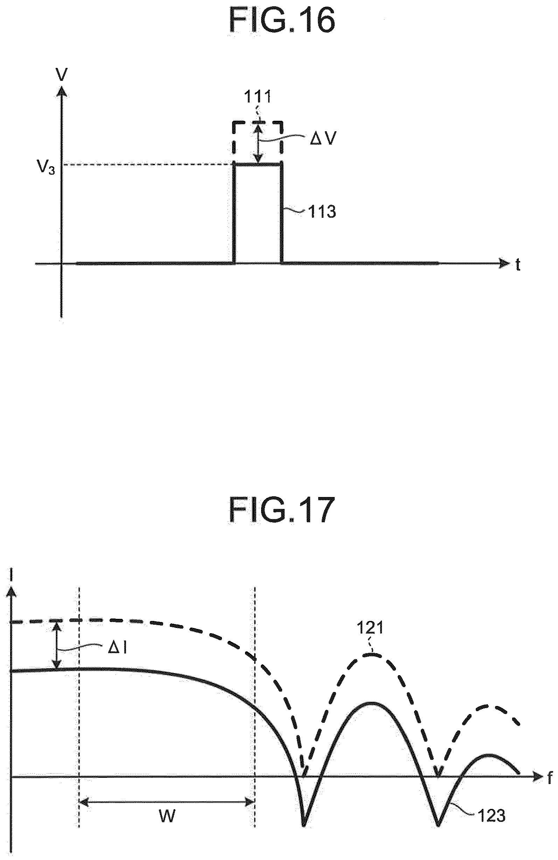

FIG. 16 is a graph schematically illustrating a waveform of a transmission drive wave according to a modification of the embodiment. In FIG. 16, in a manner similar to that in FIG. 3, a horizontal axis represents the time t, and a vertical axis represents the voltage V. A transmission drive wave 113 illustrated in FIG. 16 is a rectangular pulse having a maximum voltage (amplitude) $V_3$. The maximum voltage $V_3$ is smaller than the maximum voltage $V_1$ of the transmission drive wave 111 for the B mode illustrated in FIG. 3 by $\Delta V$.

FIG. 17 is a graph schematically illustrating a frequency spectrum of the transmission drive wave illustrated in FIG. 16. In FIG. 17, a horizontal axis represents the frequency f, and a vertical axis represents the intensity I. A spectrum 123 illustrated in FIG. 17 corresponds to such a waveform that the intensity of the spectrum 121 illustrated in FIG. 4 is uniformly reduced by $\Delta I$. The intensity difference $\Delta I$ is defined in accordance with the difference $\Delta V$ between the maximum voltage of the transmission drive wave 111 and the maximum voltage of the transmission drive wave 113.

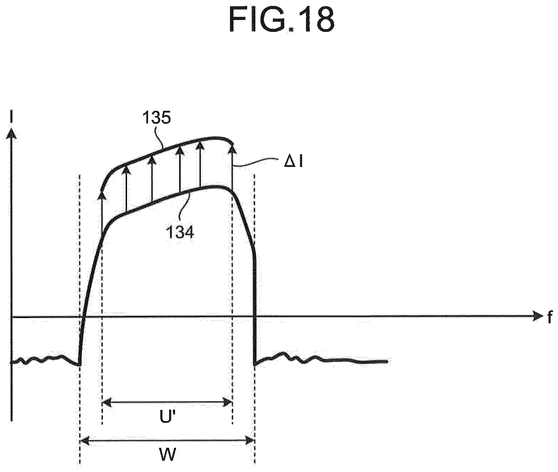

FIG. 18 is a graph illustrating a frequency spectrum calculated by the frequency analysis unit 332 when the transmission processing unit 311 generates the transmission drive wave 113 illustrated in FIG. 16, and schematically illustrating an outline of the intensity correction process performed on the frequency spectrum by the intensity correction unit 335. In the case illustrated in FIG. 18 as well, the intensity correction unit 335 performs the intensity correction on the assumption that the B mode is the reference mode. It is also illustrated in FIG. 18 that the band setting unit 334 sets a feature calculation-purpose frequency band U'.

A spectrum 134 is a frequency spectrum generated based on an echo signal reflected and returned on entirely the same condition as the spectrum 131 illustrated in FIG. 10. A correction spectrum 135 is a spectrum obtained after the intensity correction is performed on the spectrum 134 in the frequency band U' using the uniform correction amount $\Delta I$.

As is obvious from the above-mentioned modification, in the embodiment, transmission drive waves having various waveforms and frequency spectra can be generated.

Although the embodiment of the present invention has been described so far, the present invention should not be limited only by the above-mentioned embodiment. For example, the attenuation correction process of the attenuation correction unit 337 may be performed at any timing as long as it is performed before the approximation process of the approximation unit 336 and after the band setting process of the band setting unit 334, or after the approximation process of the approximation unit 336.

The present invention can also be applied to an ultrasound probe other than the above-mentioned ultrasound endoscope. As the ultrasound probe, a thin ultrasound miniature probe without an optical system may be employed. The ultrasound miniature probe is generally inserted into a biliary tract, a bile duct, a pancreatic duct, a trachea, a bronchus, a urethra, and a urinary duct, and used when peripheral organs thereof (a pancreas, a lung, a prostate, a urinary bladder, and a lymph node or the like) are examined. As the ultrasound probe, an external ultrasound probe that radiates ultrasound through a body surface of a subject may also be applied. The external ultrasound probe is generally used when an abdominal organ (a liver, a gallbladder, and a urinary bladder), a breast (in particular, a mammary gland), and a thyroid gland are examined.

In this manner, the present invention can include various embodiments in the range not departing from the technical idea described in the claims.

Reference Signs List

1 ULTRASOUND DIAGNOSIS SYSTEM
2 ULTRASOUND ENDOSCOPE
3 ULTRASOUND OBSERVATION APPARATUS
4 DISPLAY DEVICE
21 ULTRASOUND TRANSDUCER
31 TRANSMITTING AND RECEIVING UNIT
32 SIGNAL PROCESSING UNIT
33 COMPUTING UNIT
34 IMAGE PROCESSING UNIT
35 INPUT UNIT
36 CONTROL UNIT
37 STORAGE UNIT
101 CHARACTERISTIC CURVE
111, 112, 113 TRANSMISSION DRIVE WAVE
121, 122, 123, 131, 132, 134 SPECTRUM
133, 135 CORRECTION SPECTRUM
311 TRANSMISSION PROCESSING UNIT
312 RECEPTION PROCESSING UNIT
331 AMPLIFICATION CORRECTION UNIT
332 FREQUENCY ANALYSIS UNIT
333 FEATURE CALCULATION UNIT
334 BAND SETTING UNIT
335 INTENSITY CORRECTION UNIT
336 APPROXIMATION UNIT
337 ATTENUATION CORRECTION UNIT
341 ULTRASOUND IMAGE DATA GENERATING UNIT
342 FEATURE IMAGE DATA GENERATING UNIT
361 DISPLAY CONTROLLER
371 TRANSDUCER INFORMATION STORAGE UNIT
372 MODE INFORMATION STORAGE UNIT
373 INTENSITY CORRECTION INFORMATION STORAGE UNIT

**Claims**

1. An ultrasound observation apparatus for generating an ultrasound image based on an ultrasound echo obtained by an ultrasound probe, the ultrasound probe having an ultrasound transducer configured to transmit ultrasound to an observation target and receive the ultrasound reflected from the observation target, the ultrasound observation apparatus comprising:

a transmitting and receiving unit configured to: generate an electrical transmission drive wave for generating an ultrasound pulse to be transmitted by the ultrasound probe; output the transmission drive wave to the ultrasound probe; and receive an electrical echo signal from the ultrasound probe;
a frequency analysis unit configured to analyze a frequency of the echo signal to calculate a frequency spectrum of the echo signal;

a band setting unit configured to set, in accordance with the transmission drive wave, a feature calculation-purpose frequency band that is used in calculating a feature of the frequency spectrum;

an intensity correction unit configured to correct intensity of the frequency spectrum in the feature calculation-purpose frequency band set by the band setting unit, using a correction amount defined in accordance with the transmission drive wave, thereby to calculate a corrected frequency spectrum; and

an approximation unit configured to approximate the corrected frequency spectrum calculated by the intensity correction unit, in the feature calculation-purpose frequency band, thereby to extract a feature of the corrected frequency spectrum.

2. The ultrasound observation apparatus according to claim 1, wherein
the band setting unit is configured to set the feature calculation-purpose frequency band in accordance with amplitude and a frequency band of the transmission drive wave and characteristics of the ultrasound transducer of the ultrasound probe connected to the ultrasound observation apparatus.

3. The ultrasound observation apparatus according to claim 2, wherein
the band setting unit is configured to set the feature calculation-purpose frequency band by further using information on a reception depth of the ultrasound.

4. The ultrasound observation apparatus according to claim 1, wherein
a plurality of examination modes with different characteristics of transmission drive waves to be generated is settable, and
the band setting unit is configured to calculate the corrected frequency spectrum by using, as the correction amount, a difference for each frequency between the frequency spectrum of the transmission drive wave in a reference mode included in the plurality of examination modes and the frequency spectrum of the transmission drive wave generated for obtaining the electrical echo signal as a correction target.

5. The ultrasound observation apparatus according to claim 4, wherein
the plurality of examination modes includes a contrast mode for highlighting an ultrasound contrast agent to be introduced into the observation target, and
the transmission drive wave in the reference mode is a signal having a higher amplitude voltage and a wider frequency band than the transmission drive wave in the contrast mode.

6. The ultrasound observation apparatus according to claim 1, further comprising an intensity correction information storage unit configured to store intensity correction information required for correcting the intensity of the frequency spectrum, wherein
the intensity correction unit is configured to calculate the corrected frequency spectrum using the intensity correction information.

7. The ultrasound observation apparatus according to claim 2, further comprising:

a mode information storage unit configured to store information on a plurality of examination modes that is settable in the ultrasound observation apparatus and is different in characteristics of transmission drive waves to be generated; and

a transducer information storage unit configured to store information including the characteristics of the ultrasound transducer of the ultrasound probe that is connectable to the ultrasound observation apparatus, wherein
the band setting unit is configured to set the feature calculation-purpose frequency band using the information stored in the mode information storage unit and the information stored in the transducer information storage unit.

8. The ultrasound observation apparatus according to claim 1, further comprising an attenuation correction unit configured to perform an attenuation correction for reducing a contribution of attenuation occurred in accordance with a reception depth and the frequency of the ultrasound, the attenuation correction being performed before the approximation unit approximates the corrected frequency spectrum and after the band setting unit sets the feature calculation-purpose frequency band, or after the approximation unit approximates the corrected frequency spectrum.

9. The ultrasound observation apparatus according to claim 1, further comprising:

an ultrasound image data generating unit configured to generate ultrasound image data using the echo signal;

a feature image data generating unit configured to generate feature image data for displaying information related to the feature; and

a display controller configured to cause a display device connected to the ultrasound observation apparatus to display, side by side, two images corresponding to the ultrasound image data and the feature image data.

10. A method for operating an ultrasound observation apparatus for generating an ultrasound image based on an ultrasound echo obtained by an ultrasound probe, the ultrasound probe having an ultrasound transducer configured to transmit ultrasound to an observation target and receive the ultrasound reflected from the observation target, the method comprising:

a signal generating step of receiving an electrical echo signal from the ultrasound probe, by a transmitting and receiving unit configured to generate an electrical transmission drive wave for generating an ultrasound pulse to be transmitted by the ultrasound probe to output the transmission drive wave to the ultrasound probe;

a frequency analysis step of analyzing, by a frequency analysis unit, a frequency of the echo signal to calculate a frequency spectrum of the echo signal;

a band setting step of setting, by a band setting unit, a feature calculation-purpose frequency band that is used in calculating a feature of the frequency spectrum, in accordance with the transmission drive wave;

an intensity correction step of correcting, by an intensity correction unit, intensity of the frequency spectrum in the feature calculation-purpose frequency band, using a correction amount defined in accordance with the transmission drive wave, thereby to calculate a corrected frequency spectrum; and

an approximation step of approximating the corrected frequency spectrum in the feature calculation-purpose frequency band, thereby to extract a feature of the corrected frequency spectrum.

11. A program for operating an ultrasound observation apparatus for generating an ultrasound image based on an ultrasound echo obtained by an ultrasound probe, the ultrasound probe having an ultrasound transducer configured to transmit ultrasound to an observation target and receive the ultrasound reflected from the observation target, the program causing the ultrasound observation apparatus to execute:

a signal generating step of receiving an electrical echo signal from the ultrasound probe, by a transmitting and receiving unit configured to generate an electrical transmission drive wave for generating an ultrasound pulse to be transmitted by the ultrasound probe to output the transmission drive wave to the ultrasound probe;

a frequency analysis step of analyzing, by a frequency analysis unit, a frequency of the echo signal to calculate a frequency spectrum of the echo signal;

a band setting step of setting, by a band setting unit, a feature calculation-purpose frequency band that is used in calculating a feature of the frequency spectrum, in accordance with the transmission drive wave;

an intensity correction step of correcting, by an intensity correction unit, intensity of the frequency spectrum in the feature calculation-purpose frequency band, using a correction amount defined in accordance with the transmission drive wave, thereby to calculate a corrected frequency spectrum; and

an approximation step of approximating the corrected frequency spectrum in the feature calculation-purpose frequency band, thereby to extract a feature of the corrected frequency spectrum.

# FIG.1

1

ULTRASOUND OBSERVATION APPARATUS ⌐3

ULTRASOUND ENDOSCOPE ⌐2
- ULTRASOUND TRANSDUCER ⌐21

TRANSMITTING AND RECEIVING UNIT ⌐31
- TRANSMISSION PROCESSING UNIT ⌐311
- RECEPTION PROCESSING UNIT ⌐312

SIGNAL PROCESSING UNIT ⌐32

INPUT UNIT ⌐35

STORAGE UNIT ⌐37
- TRANSDUCER INFORMATION STORAGE UNIT ⌐371
- MODE INFORMATION STORAGE UNIT ⌐372
- INTENSITY CORRECTION INFORMATION STORAGE UNIT ⌐373

CONTROL UNIT ⌐36
- DISPLAY CONTROLLER ⌐361

COMPUTING UNIT ⌐33
- AMPLIFICATION CORRECTION UNIT ⌐331
- FREQUENCY ANALYSIS UNIT ⌐332
- FEATURE CALCULATION UNIT ⌐333
  - BAND SETTING UNIT ⌐334
  - INTENSITY CORRECTION UNIT ⌐335
  - APPROXIMATION UNIT ⌐336
  - ATTENUATION CORRECTION UNIT ⌐337

IMAGE PROCESSING UNIT ⌐34
- ULTRASOUND IMAGE DATA GENERATING UNIT ⌐341
- FEATURE IMAGE DATA GENERATING UNIT ⌐342

DISPLAY DEVICE ⌐4

EP 3 295 876 A1

# FIG.2

# FIG.3

# FIG.4

# FIG.5

# FIG.6

# FIG.7

# FIG.8

# FIG.9

SHALLOW ────────────────────────────────────► DEEP

$Z^{(k)}_0$

D

$\underline{SR_k}$

$Z^{(k)}_{max}$

$F_1$

$F_2$

$F_3$

· · · · · ·

$F_{K-1}$

$F_K$

EP 3 295 876 A1

# FIG.10

# FIG.11

# FIG.12

# FIG.13

# FIG.14

START

RECEIVE ECHO SIGNAL — S1

PERFORM PREDETERMINED RECEPTION
PROCESS ON ECHO SIGNAL — S2

GENERATE ULTRASOUND IMAGE DATA — S3

PERFORM AMPLIFICATION CORRECTION — S4

PERFORM FREQUENCY ANALYSIS — S5

SET FREQUENCY BAND FOR CALCULATING
FEATURE — S6

PERFORM INTENSITY CORRECTION — S7

EXTRACT TEMPORARY FEATURE — S8

PERFORM ATTENUATION CORRECTION ON
TEMPORARY FEATURE — S9

GENERATE FEATURE IMAGE DATA — S10

DISPLAY ULTRASOUND IMAGE AND FEATURE
IMAGE SIDE BY SIDE ON DISPLAY DEVICE — S11

END

# FIG.15

PERFORM FREQUENCY
ANALYSIS

$k=k_0$ — S21

$Z^{(k)}=Z^{(k)}_0$ — S22

OBTAIN SAMPLE DATA GROUP — S23

APPLY WINDOW FUNCTION — S24

S25

IS SAMPLE DATA GROUP
NORMAL?

NO

S26

INSERT ZERO DATA TO
COVER SHORTFALL

YES

PERFORM FFT COMPUTATION — S27

$Z^{(k)}=Z^{(k)}+D$ — S28

S29

$Z^{(k)}>Z^{(k)}_{max}$ ?

NO

YES

$k=k+1$ — S30

S31

NO

$k>k_{max}$ ?

YES

RETURN

26

# FIG.16

# FIG.17

# FIG.18

## INTERNATIONAL SEARCH REPORT

| | International application No. |
|---|---|
| | PCT/JP2016/063441 |

**A. CLASSIFICATION OF SUBJECT MATTER**
*A61B8/12*(2006.01)i

According to International Patent Classification (IPC) or to both national classification and IPC

**B. FIELDS SEARCHED**

Minimum documentation searched (classification system followed by classification symbols)
A61B8/12

Documentation searched other than minimum documentation to the extent that such documents are included in the fields searched
| Jitsuyo Shinan Koho | 1922–1996 | Jitsuyo Shinan Toroku Koho | 1996–2016 |
| Kokai Jitsuyo Shinan Koho | 1971–2016 | Toroku Jitsuyo Shinan Koho | 1994–2016 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | WO 2012/063928 A1 (Olympus Medical Systems Corp.), 18 May 2012 (18.05.2012), paragraphs [0030] to [0074]; fig. 1 to 16 & JP 2013-166059 A & US 2013/0035594 A1 paragraphs [0037] to [0082]; fig. 1 to 16 & EP 2599440 A1 & CN 103200876 A | 1–11 |
| A | WO 2012/063929 A1 (Olympus Medical Systems Corp.), 18 May 2012 (18.05.2012), paragraphs [0029] to [0041], [0068] to [0070]; fig. 2, 3, 7 to 10 & JP 5307939 B2 & US 2013/0030296 A1 paragraphs [0031] to [0044], [0071] to [0073]; fig. 2, 3, 7 to 10 & EP 2599441 A1 & CN 103153195 A | 1–11 |

☐ Further documents are listed in the continuation of Box C.   ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 06 June 2016 (06.06.16) | 14 June 2016 (14.06.16) |

| Name and mailing address of the ISA/ Japan Patent Office 3-4-3,Kasumigaseki,Chiyoda-ku, Tokyo 100-8915,Japan | Authorized officer |
|---|---|
| | Telephone No. |

Form PCT/ISA/210 (second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012011414 A

- JP 4820494 B